# EUROPEAN PATENT APPLICATION

(11) **EP 3 915 434 A1**
(43) Date of publication of application: **01.12.2021**
(21) Application number: 20176965.0
(22) Date of filing: 28.05.2020
(51) Int. Cl.: A46B 15/00

(54) **ORAL CARE DEVICE AND METHOD**

(71) Applicant: Koninklijke Philips N.V., 5656 AG Eindhoven (NL)
(72) Inventor: JOHNSON, Mark Thomas, 5656 AE Eindhoven (NL); GOTTENBOS, Bart, 5656 AE Eindhoven (NL); VAN HULTEN, Maaike Cornelia Johanna Wilhelmina, 5656 AE Eindhoven (NL); GERHARDT, Lutz Christian, 5656 AE Eindhoven (NL)
(74) Representative: Philips Intellectual Property & Standards

(57) **Abstract**

An oral care device (8) for use in aiding delivery of a substance for an oral treatment purpose within the mouth. The device comprises a field generator (16) which is adapted to generate a field (20) for exerting a force on particles (20) susceptible to the field to guide movement of the particles to a desired location. When operated in the mouth, this allows oral treatment particles to be quickly, safely and efficiently distributed within the mouth to the correct locations for performing the oral treatment function.

## Description

### FIELD OF THE INVENTION

The present invention relates to an oral care device, in particular a device for deploying a cleaning or treatment agent in the mouth.

### BACKGROUND OF THE INVENTION

To aid in oral cleaning or treatment, active agents such as fluoride, whitening agents, mouthwash or antimicrobially effective particles are commonly used.

However, for most effective utilization of these agents, there is a need for homogeneous and preferably fast delivery of the agents across the regions of the mouth where they are required. For example, whitening agents need to be homogeneously spread across surfaces of the teeth, and antimicrobial particles need to be spread evenly across portions of the gums or between the teeth. However, delivering the agents to the parts of the mouth where they are needed is difficult and inefficient, which reduces their efficacy and causes inconvenience for a user.

Radio-frequency (RF) electromagnetic emissions can also be used to provide a cleaning function in the oral cavity. In particular, an oral cleaning device can include a cleaning unit, such as a head or mouthpiece portion, for insertion into an oral cavity of a user, which cleaning unit portion includes two or more electrodes coupled to an RF signal generator. The signal generator drives the electrodes with an RF signal which causes RF radiation to be emitted around and between the electrodes.

According to US10201701B2, when the RF field interacts with surfaces of the teeth and gums, it provides a cleaning function by loosening the bonds between impurities and the surfaces in the mouth. In particular, RF fields generated in this way can remove dental plaque, and also dental calculus. Staining of teeth can also be reduced.

US10201701B2 describes a prior art electric toothbrush. The toothbrush comprises a platen, an RF generator, two RF electrodes, and a dielectric barrier situated between the two RF electrodes in the form of a silicone strip. The toothbrush also includes bristles. The dielectric barrier has a height which extends up to the level of the distal tips of the brush bristles. The barrier forces RF waves transmitted between the electrodes to travel over the top of the barrier, thus reaching the area where the bristles engage with the surfaces of the teeth and gums in use.

### SUMMARY OF THE INVENTION

The invention is defined by the claims.

According to examples in accordance with an aspect of the invention, there is provided an oral care device comprising:
a field generator adapted to generate a first electric, electromagnetic, and/or magnetic field in a space;
the field for imparting a force upon particles susceptible to the field, for guiding movement of the particles to a desired one or more locations in the space,
wherein the field generator is for operation in a mouth of a user, for guiding movement of the particles to a desired one or more locations in the mouth.

Embodiments of the invention are based on manipulating the position or distribution of particles comprising oral treatment agents within the mouth using electric, magnetic, or electromagnetic fields.

The field generator creates a field with a certain field pattern within a space in the mouth. The field pattern encompasses field strength gradients or field lines which define a pattern of force paths through the space, which guide movement of particles sensitive to such fields to a particular location. The field is for example adapted to guide particles to move from one part of the mouth to another.

The field may be for guiding particles to a desired location or a desired pattern of locations. The field may be for guiding particles to adopt a desired spatial distribution.

This is an efficient, safe and reliable means of delivering oral treatment agents to the required regions in the mouth.

In some examples, the device may comprise a support body, the support body for receipt in an oral cavity of a user, wherein the field generator is adapted to generate a field for guiding particles susceptible to the field over, across or alongside one or more surfaces of the support body.

For example, the field generator may be carried by or integrated in the support body.

The field may be generated in a space which extends across, along or alongside at least a portion of said surface of the support body.

The particles may be in contact with the support body when they are being guided, or may be suspended above or below the surface, e.g. located in a volume around the surface of the support body, e.g. suspended in a fluid. The particles may be guided along a path parallel the surface. The particles may be guided to a desired one or more locations in the mouth.

The particles may be electrically polarized or polarizable particles, or magnetic particles, or electrically charged particles. The particles manipulated by the field may themselves be particles of an oral treatment agent, or may be carrier particles, which contain within them a treatment agent to be deployed. For example, the particles may be capsules for containing therein a treatment agent.

The field generator can be an actively driven field generator (e.g. comprising coils or electrodes driven with a current or voltage to generate a field) or a passive field generator (comprising for example an arrangement of permanent magnets defining a field pattern which guides particles).

For example, in the active case, the field generator may be adapted to generate said first field responsive to an input control signal.

The device may further comprise a controller adapted to generate the control signal, to control generation of the electric, electromagnetic and/or magnetic field in the space, the field for guiding movement of the particles to a desired one or more locations in the mouth. The field pattern generated by the field generator may be dependent upon the control signal and/or upon the geometry of the field generator (e.g. the geometry or arrangement of electrodes or coils).

The control signal may be a drive signal for driving current or charge through conductive elements to generate the field. The drive signal may be a DC or an AC drive signal.

There are different ways of inducing forces on the susceptible particles, depending upon the particular electric or magnetic properties of the particles the device is to be used with.

According to one advantageous set of embodiments, the field generator and/or control signal (in the active field generator case) may be configured to generate a (spatially) non-uniform field (non-uniform field strength) having a field strength gradient in one or more directions, for guiding the particles along a direction of the gradient.

A non-uniform field such as this can be used to move electrically or magnetically polarizable or polarized particles for example. A polarized or polarizable particle will move along the direction of the field strength gradient due to the non-uniform force experienced at each pole of the particle's electric or magnetic dipole. Thus the field can be configured so as to exhibit field strength gradients which define one or more gradient paths leading toward locations where the particles are desired to be delivered.

For example, according to a particular set of embodiments, the field generator and/or control signal may be configured to generate: a spatially non-uniform electric or electromagnetic field, having a field strength gradient in one or more directions, for guiding electrically polarizable particles along a direction of the gradient and/or for guiding charged particles along a direction of field lines (along a vector direction of the field).

The electric field may be static or alternating. It may be an alternating electromagnetic field.

A non-uniform electric or electromagnetic field can be used to move electrically polarizable or polarized particles by means of the dielectrophoresis (DEP) effect.

Since the movement is dependent on a vector direction of a gradient in field strength, not a direction of the field lines, the movement in a given direction is independent of the vector direction of the field at a given point.

Any electric field (uniform or non-uniform) can also be used to move electrically charged particles along field lines. The benefit therefore of a non-uniform field is that it can be used to move both electrically charged and electrically polarizable particles, one along field gradient directions, the other along vector directions of the field itself.

One advantageous set of embodiments proposes to use electrically polarizable capsules having oral treatment agents contained therein, the electrically polarizable capsules providing the particles susceptible to the field. These capsules can be moved by means of the non-uniform electrical field to the desired locations in the mouth. The active agent therein may then be deployed at those locations. This might happen without further interaction from the device, e.g. the capsules are water soluble and break down due to interaction with saliva, or it may be triggered by an activation stimulus generated by the device.

An analogous effect to the DEP effect can be induced for magnetizable particles by generating a non-uniform magnetic field, to induce movement along a direction of a magnetic field strength gradient.

There are also different options for the structure of the field generator.

By way of example, the field generator may comprise one or more conductive elements, electrically coupled to the controller. The control signal may be a drive signal for electrifying the elements, e.g. supplying a voltage or current.

The conductive elements may be electrodes for example, or may be coils or wires.

A control signal may be provided in the form of an electrical drive signal in this set of embodiments. The control signal provides a voltage or current supply for example. The signal energizes the conductive elements.

With reference to the field generator, there are two main options for the field that is generated: an electric field and/or a magnetic field. In practice, an electromagnetic field might be generated, but with a different one of the electric or magnetic field dominant in each case for example. The electric or magnetic field may be a static field or may be an alternating field in different examples. The field generator can be an active field generator or passive field generator (e.g. comprising an arrangement of permanent magnets).

Options relating to generation of an electric field will first be discussed.

According to one or more embodiments, the one or more conductive elements may include at least one pair of electrodes separated by a space (extending from one electrode to the other), for generating an electric field in the space. The field may be for imparting a force on the particles to guide movement of the particles through the space, form one point to another. The particles thus may in general move in a direction toward one of the electrodes and away from the other of the electrodes.

As discussed above, in some examples, the field generator and/or control signal may be configured to generate a non-uniform electric field, having a field strength gradient in one or more directions, for guiding polarizable particles along a direction of the gradient. Electrically charged particles that are present will follow a direction of the field lines.

To facilitate the (spatially) non-uniform electric field, the one or more conductive elements may include at least one pair of electrodes separated by a space, and wherein the electrodes differ in size. Thus an asymmetric pair of electrodes is provided. By way of example, one electrode may have a length which spans a longer distance than the other along a direction perpendicular their separation. This results in a field which is more spatially spread on one side of the separation space than the other, with the result that there is a field strength gradient toward the smaller electrode, and this with the result that there is a net force toward the smaller electrode. This will become clear in further explanations to follow.

By way of example, each electrode of the pair may have a respective electrically chargeable or active area for generating said electric field in the space between the pair when electrically charged, and wherein the electrically chargeable or active areas of the two electrodes differ in size. Thus, the parts of the electrodes which become charged and contribute to the field may differ in area between the electrodes. They may additionally or alternatively differ in dimensional extension along a direction perpendicular to a direction of the separation between the electrodes.

In accordance with one or more embodiments, the field generator and/or control signal may be configured to generate a magnetic field (uniform or non-uniform).

The field generator may comprise one or more electrically conductive elements for carrying an electrical current, and wherein a controller is adapted to supply a drive signal to the conductive elements to drive a current through the elements to generate a magnetic field in the space. The field maybe for guiding magnetic particles along a direction of field lines of the field.

The conductive elements may be electrodes, wires, or coils for example.

Additionally or alternatively, the field generator may comprise a permanent magnet arrangement.

Magnetic particles will be guided along the vector direction of the field which is generated.

An advantageous set of embodiments proposes to use Catalytic Antimicrobial Robots (CAR) as the treatment agent to be deployed by the device. These exploit iron oxide nanoparticles with dual catalytic magnetic functionality to (i) generate bactericidal free radicals, (ii) break down the biofilm exopolysaccharide (EPS) matrix, and (iii) remove the fragmented biofilm debris via magnetic field gradient-driven robotic assemblies in a controlled manner, preventing biofilm regrowth.

CARs are magnetic particles and thus can be induced to move using a magnetic field. They may move along the directions of magnetic field lines for example.

As discussed above, an RF alternating field can be generated and used to provide an oral cleaning and/or treatment function.

In accordance with one or more embodiments, the controller may be adapted, in at least one mode, to generate a control signal configured to cause the field generator to generate an RF alternating (electromagnetic) field, for performing an oral cleaning function when the electrodes are received in an oral cavity of a user.

For example, the field generator may comprise one or more conductive elements, and the controller may supply a control signal in the form of an RF alternating drive signal (e.g. an alternating current or potential) to or between the one or more conducive elements.

In this way, the same components of the field generator which are used to manipulate the positioning of the particles is also used to generate an RF field for cleaning.

In some examples, the first field (for guiding movement of the particles) may itself be an RF alternating field. Thus, the field which induces movement of the particles to the desired location may advantageously also performs an RF oral cleaning function due to its RF frequency alternation. The RF cleaning function may occur simultaneously to the movement function.

In further examples, the first field (for guiding movement of the particles) may not itself be an RF field, or may be an RF field, but does not perform an RF cleaning function.

In accordance with one or more embodiments, the controller may be selectively operable in at least two different modes:
a particle delivery mode in which the controller supplies a control signal to the field generator for generating a field for guiding particles susceptible to the field to a desired one or more locations in the space; and
a cleaning mode in which the controller supplies a control signal to the field generator for generating an RF field in the space for performing an oral cleaning function.

The field generated in the particle delivery mode may be an RF field, or may not be an RF field (e.g. it may be a DC static field). In some cases, the field generated in the particle delivery mode may be an RF field, having a different RF frequency to the RF field of the cleaning mode.

In accordance with one or more embodiments, the device may be configured with functionality of triggering an activation of delivered particles. This activation may be a chemical or physical reaction process, or a process of breaking down particles to trigger release of their contents. This step may be performed subsequent to moving the particles to the desired locations in the mouth for example.

Thus in accordance with one or more embodiments, the device may further comprise a particle activator adapted to generate a physical stimulus for stimulating an activation event of the susceptible particles. The particle activator is composed of the same or different components to the field generator. Thus, the particle activator may be provided by the field generator in some example, or may be a formed by a separate set of one or more components.

The stimulus may, by way of non-limiting example, include at least one of: a generated field (electrical and/or magnetic stimulus), a heat stimulus, and an acoustic stimulus.

In accordance with one or more embodiments, the device may further comprise a cleaning stimulus generator, for generating a physical stimulus for stimulating breakdown or removal of a biofilm from a surface within an oral cavity of a user. Again, the cleaning stimulus generator may be composed of the same or different components to the field generator.

In accordance with one or more embodiments, the controller may be adapted, in at least one control mode, to execute a particle delivery control scheme.

The control scheme comprises at least a first step of supplying the control signal to the field generator to guide the particles to the desired location. This is a particle deployment step.

The control scheme further comprises at least a second (subsequent) step of supplying a second control signal to the particle activator to create a stimulus for activating the particles. This is a particle activation step.

Optionally, the control scheme may further comprise at least a third step of supplying a third control signal to the cleaning stimulus generator to generate a cleaning stimulus for stimulating breakdown or removal a biofilm residue on an oral surface (e.g. at the location of the activated particle(s)).

As discussed above, the field generator can be an active field generator, driven by a control signal, or a passive signal generator.

In accordance with one set of embodiments for example, the field generator may be a passive field generator comprising a permanent magnet arrangement. The field generator generates a static magnetic field in a space based on use of the permanent magnetic field arrangement, the static magnetic field for guiding magnetic particles along a direction of field lines of the field, or along a direction of field gradients of the field.

In accordance with one or more embodiments, the field generator may be carried by or integrated in a support body, the support body for receipt in an oral cavity of a user, for generating a field in the oral cavity for guiding particles to a desired one or more locations in the oral cavity. The field may be for guiding particles across, over or parallel one or more surfaces, either in contact with the surface or suspended above or below the surface to desired locations in the mouth.

In particular examples, the permanent magnet arrangement may comprise a first and second permanent magnet portion, the first and second magnet portions having opposing magnetic poles, and being spaced apart such that a magnetic field is provided in a region between the portions, the arrangement for guiding magnetic particles in one or more directions through said region.

For instance, the generated magnetic field may be shaped or configured to guide particles toward one of the magnetic portions. In other examples, the field may be for guiding particles traverse the magnetic portions.

In an advantageous set of examples, the field generator may further comprise one or more magnetic field shaping elements disposed in the space between the first and second portions, each arranged to become magnetized by a magnetic field between the first and second magnetic portions, for shaping a magnetic field in a region between the first and second magnetic portions.

The field shaping elements may each comprise a body or element formed of or comprising a magnetic material, for example a ferromagnetic material. When the element becomes magnetized, it alters the shape of the field lines and/or the field gradient within the region between the first and second magnetic portions. For example, it emits a secondary magnetic field. The resultant magnetic field defines for example one or more vector field paths along which magnetic particles brought into the field are induced to follow.

In an advantageous set of embodiments, the device may be in the form of a mouthpiece unit comprising a pair of protruding ridge members, protruding upwards from an intermediate separation member (e.g. biting surface), and the protruding ridges defining a tooth receiving channel between them. The first and second magnetic portions may be located on opposite sides of the channel, for example incorporated in, or adjacent to, different respective ones of the two ridges. The field shaping element is preferably incorporated in the intermediate separation member (e.g. biting surface) but can be also part of any other element provided a static magnetic field acts across the field shaping element.

The field may be shaped to guide movement of particles along the tooth receiving channel, for example along or over the intermediate separation member (e.g. the biting surface), or toward one or both of the protruding ridges.

In accordance with at least one set of embodiments, the support body may be in the form of a mouthpiece unit, and the mouthpiece arranged to fit into the mouth of a user with surfaces of the mouthpiece overlying or facing surfaces of the mouth or teeth.

The body of the mouthpiece may describe a U-shaped contour for example. It may comprise an upper and lower tooth-receiving channel, for receiving the upper and lower rows of teeth therein, with an intermediate separation element between the channels which defines a biting surface.

A further aspect of the invention also provides an oral cleaning system, comprising:
a device in accordance with any example or embodiment outlined above or described below, or in accordance with any claim of this application, and
a substance for delivery in the mouth, the substance comprising particles susceptible to the field.

The system may comprise a reservoir unit adapted to releasably dock or couple with the cleaning device for loading or charging the device with particles for delivery during use. A docking station may be provided which comprises the reservoir of particles, and the device adapted to releasably mechanically couple with the docking station to be refilled with particles for delivery.

The device may include for example a local reservoir or storage chamber for receiving and storing particles for deployment. There may be a flow path between the local reservoir and one or more surfaces of the device, e.g. surfaces which, when the device is received in the mouth in use, face dental or oral surfaces of the user. The particles may be delivered to different areas in the mouth by controlling generation of a field which moves the particles across or along or parallel the one or more surfaces, either in contact with the surface or suspended above or below the surface to desired locations in the mouth.

According to one set of advantageous embodiments, the substance may comprise (electrically) polarizable capsules containing a treatment agent for delivery.

According to a further set of advantageous embodiments, the substance may comprise magnetizable or (ferro-, dia-, para-)magnetic particles such as ferrite based catalytic antimicrobial robots (CARs).

A further aspect of the invention also provides a method for delivering particles for a treatment function within an oral cavity of a user, the method comprising:
delivering a control signal to a field generator, to control generation by the field generator of an electric and/or magnetic field, the field for imparting a force upon particles susceptible to the field, for guiding movement of the particles to a desired one or more locations in an oral cavity of a user when the field generator is received in said oral cavity.

The method may be performed with the field generator in the oral cavity (mouth) of a user.

Examples in accordance with a further aspect of the invention provide a computer program product comprising computer program code, the computer program code being executable on a processor or computer
wherein, when the processor or computer is operatively coupled with a field generator, the code is configured to cause the processor to perform a method in accordance with any example or embodiment outlined above or described below, or in accordance with any claim of this application.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an example oral care device according to one or more embodiments;
Fig. 2 shows an example oral care device in the form of a mouthpiece;
Fig. 3 illustrates a cross-sectional view of an example oral care device in the form of a mouthpiece unit;
Fig. 4 shows an example oral care device wherein a non-uniform field is generated;
Fig. 5 illustrates movement of a polarizable or polarized particle in a non-uniform electric field;
Fig. 6 illustrates a further example oral care device with a non-uniform field;
Fig. 7 illustrates a further example oral care device;
Fig. 8 illustrates a further example oral care device with a non-uniform field;
Fig. 9 illustrates a further example oral care device with a non-uniform field;
Fig. 10 illustrates a further example oral care device in which a magnetic field is generated;
Fig. 11 illustrates a further example oral care device in which a magnetic field is generated;
Figs. 12-13 illustrate an example device according to one or more embodiments with a passive field generator which comprises an arrangement of permanent magnets; and
Figs. 14-15 illustrate guidance of particles using a magnetic field shaped using magnetic field shaping elements.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an oral care device for use in aiding delivery of a substance for an oral treatment purpose within the mouth. The device comprises a field generator which is generates a field for exerting a force on particles susceptible to the field to guide movement of the particles to a desired location or pattern of locations. When operated in the mouth, this allows oral treatment particles to be quickly, safely and efficiently distributed within the mouth to the correct locations for performing the oral treatment function.

The device may include a device body (e.g. support body) for being received in the mouth, and having one or more surfaces which face oral or dental surfaces when received in the mouth, and wherein the field generator is adapted to generate a field which guides particles over or across or parallel to one or more of said surfaces to a desired set of one or more locations in the mouth, or to a pattern of locations, or into a desired spatial distribution.

The delivered oral treatment substance can include, by way of non-limiting example, active agents such as fluoride, whitening solutions, mouthwash and specialized toothpaste, and gel for mouthpiece devices. The delivered agent can also according to one set of advantageous embodiments, include catalytic antimicrobial robots (CARs). Any other substance for an oral cleaning or treatment purpose can also be considered.

The particles susceptible to the field may be particles of the treatment agent itself or capsules for carrying or transporting the agent, as will be explained further below.

The field generator may be an active field generator (driven by a control or drive signal to generate a field) or a passive field generator (e.g. comprising one or more permanent magnets). A set of embodiments comprising active field generators will first be described.

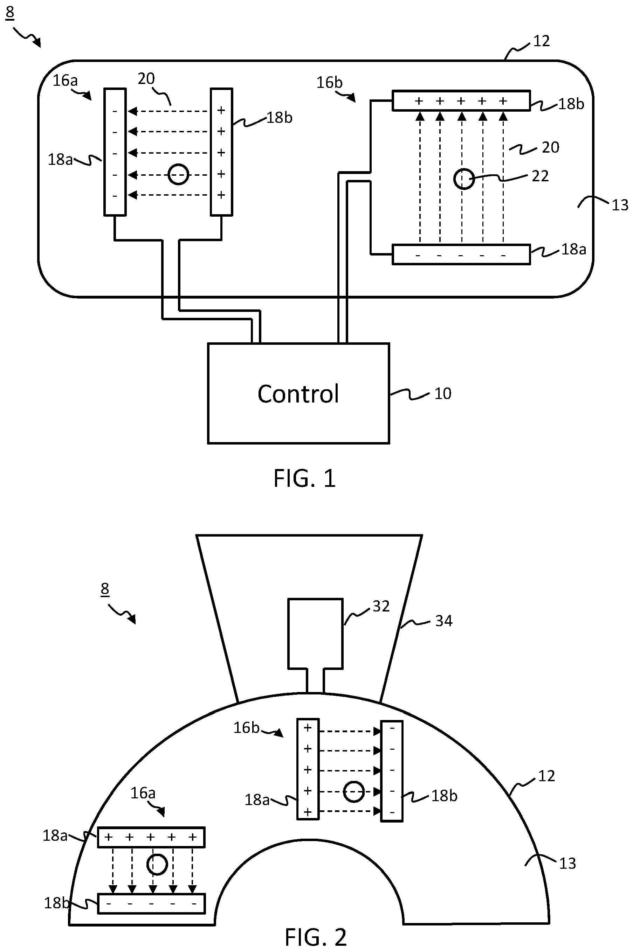

Fig. 1 schematically illustrates components of a first example oral care device according to one or more embodiments.

The device 8 comprises a field generator 16 adapted to generate a first electric and/or magnetic field 20 in a space responsive to an input control signal.

The device further includes a controller 10 adapted to generate the control signal, to control generation of an electric and/or magnetic field 20 in the space. The field is for imparting a force upon particles 22 susceptible to the field, for guiding movement of the particles to a desired location in the space.

The field generator 16 is for operation in a mouth of a user, for guiding movement of the particles to a desired location in the mouth.

In this example, the field generator comprises two field generation arrangements or portions 16a, 16b, each comprising a first 18a and second 18b conductive body which are arranged to receive the control signal from the controller 10. Each generates a respective local field 20 between the respective conductive bodies, for guiding movement of particles 22 in a local area. However, this is not essential and in other examples, a single field generation arrangement could be provided, arranged to generate a field over a single area.

In this example, the device 8 comprises a device support body 12 which carries or integrates the field generator 16a, 16b. For example the field generator is carried on a support surface 13 of the support body or is integrated beneath the support surface. The controller 10 may also be carried by or integrated in the support body 12, or it may be separate from the support body and electrically coupled or coupleable with the field generator 16 of the support body. For example, in some cases, the device 8 may further comprise a handle or body portion to which the support body 12 couples or attaches, and which integrates the controller 10.

In the example illustrated in Fig. 1, each field generation arrangement 16a, 16b of the field generator 16 comprises a respective pair of electrodes 18ab 18b, separated by a space, and arranged to receive a voltage supply from the controller 10 causing charging of the electrodes. This induces an electric field 20 between the electrodes.

However, as will be explained in other examples below, the field generator 16 may in alternative cases be arranged to generate a magnetic field. In this case the field generator may comprise one or more conductive bodies 18 which are arranged to carry a current supplied by the controller 10, e.g. one or more wires or coils.

Thus, the control signal may be a drive signal for driving current or charge through conductive elements 18 to generate the field. The drive signal may be a DC or an AC drive signal. The field may be a static (DC) field, or an alternating (AC) field.

When the field 20 is generated by the field generator 16, the particles 22 susceptible to the field are guided to move due to a force induced on the particles by the field. For example, the particles may be guided to move along or across the surface 13 of the support body 12 in some cases to thereby transport them to the required location in the mouth. The support surface may be exposed in the mouth in some examples. It may face dental or oral tissue surfaces in the mouth so that movement of the particles across the support surface 13 guides the particles to a desired location in the mouth.

Thus embodiments of the invention are based on manipulating the distribution of particles comprising oral treatment agents within the mouth using electric, magnetic, or electromagnetic fields.

In different examples, the particles 22 may be electrically or magnetically polarized or polarizable particles, or magnetic particles, or electrically charged particles. The particles manipulated by the field may themselves be particles of an oral treatment agent, or may be carrier particles, which contain within them a treatment agent to be deployed. For example, the particles may be capsules for containing therein a treatment agent.

In some examples, the control signal generated by the controller 10 may be dynamically varied as a function of time, to dynamically control a force exerted on susceptible particles 22. A drive scheme of the control signal may be varied over time.

The control signal may be varied over time to dynamically configure a shape or pattern of the field 20, to thereby configure the force effect for example.

There are different options for the form and structure of the device 8 depending upon the application. For example, the invention can be implemented in a range of different oral care devices such as mouthpiece devices, toothbrushes, interdental cleaning devices, flossing devices, and oral irrigator devices.

One example implementation in the form a mouthpiece device is shown in Fig. 2. The mouthpiece unit comprises an arcuate support body 12 with a support surface 13. The arcuate shape permits the mouthpiece to be received in an oral cavity of the user. It may be shaped to conform to the geometry or contours of a user's mouth (e.g. a specific user or a typical user). It may have upper and lower surfaces. The conductive elements of the field generator 16 may be carried by the support surface 13 of the support body 12 or integrated beneath the support surface.

As in the example of Fig. 1, in this example the field generator 16 comprises a plurality of pairs of electrodes 18a, 18b, with the controller applying a potential difference between each pair to thereby induce an electric field in a space between the electrodes. The controller 10 is not shown in Fig. 2, for ease of illustration. Each pair of electrodes forms a respective field generator portion, or field generation arrangement 16a, 16b which generates a field at a local region of the mouthpiece. Each field generation arrangement 16a, 16b thus effectively allows a respective particle collection location to be defined, since particles will collect adjacent one of each of the electrodes of each pair. Thus particles can be guided to a plurality of different defined locations across the support body 12 surface 13. When the device is received in the mouth, this thereby pulls or pushes the particles to particular regions in the mouth.

The device 8 may further comprise a handle portion 34, coupled to the support body 12. The support body may form a mouth-receivable portion, e.g. a mouthpiece. The handle may be fixedly attached to the support body 12, or it may be releasably coupleable with the support body. The handle may house the controller 10 in some examples.

There may be further included a reservoir 32 of particles comprising a store or source of the oral treatment agent. This may be integrated in the handle 34 portion of the device for example. A flow path may be defined between the reservoir and the support body which permits particles to be received from the reservoir 32 onto one or more surfaces 13 of the support body. The particles may then be distributed from there across the support surface 13 to different locations in the mouth.

The particle reservoir 32 shown in Fig. 2 may be a local reservoir of particles, comprised by the device. In further examples, an external reservoir may additionally or alternatively be provided, for instance in the form of a bottle, a tube, or any other receptacle. The reservoir may be provided in the form of a docking station in some examples. In each case, the reservoir may be adapted to releasably dock or couple with the device 8 for loading or charging the device 8 with particles to be distributed in the mouth. This may be done each time the device is used, and the particles may be received for example directly onto one or more surfaces 13, ready to be deployed. In further examples, an external reservoir may be adapted to releasably dock or couple with a local reservoir 32 of the device, to re-fill the local reservoir with particles.

A reservoir of particles (e.g. an external, removable reservoir), in combination with the support body 12, controller 10 and field generator 16 may form an oral treatment system.

Although Fig. 2 shows the mouthpiece comprising an electric field generator, in further examples, the mouthpiece may comprise a field generator 16 adapted to generate a magnetic field.

The device 8 of Fig. 2 is in the form of a mouthpiece unit. The mouthpiece may simply comprise a flat planar support body 12, e.g. a platen, with a flat upper support surface 13, the support body forming for example a biting plate that teeth can rest on.

In further examples, the mouthpiece unit may have a shaped profile configured to conform to dental or oral features of the mouth.

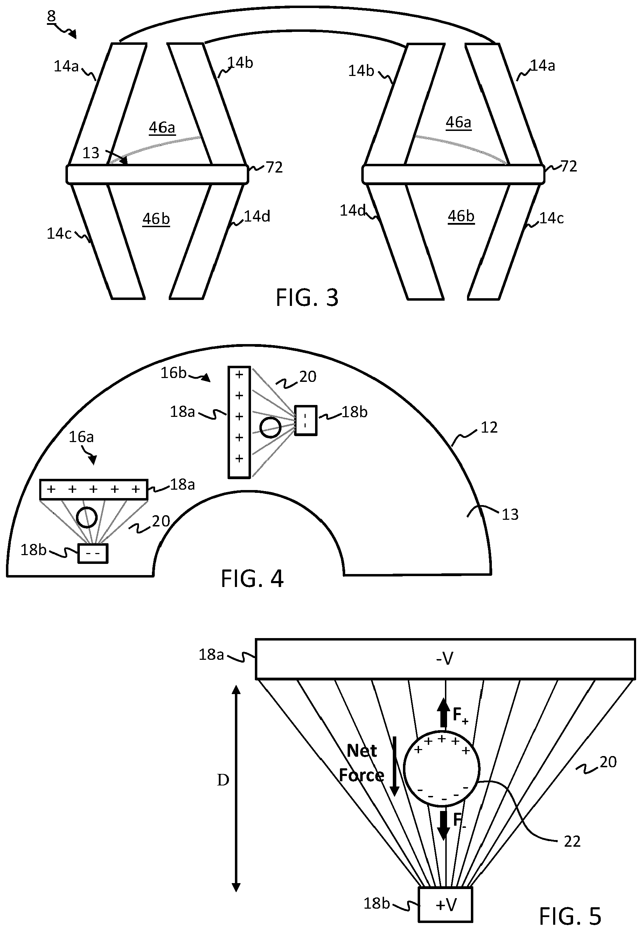

For example, Fig. 3 shows one example in which the device 8 comprises a mouthpiece unit having a structure which defines an upper tooth-receiving channel 46a and a lower tooth receiving channel 46b, the upper channel defined between and bounded by first 14a and second 14b protruding ridges, and the lower channel defined between and bounded by third 14c and fourth 14c protruding ridges. The first and second ridges protrude outward from an upper surface 13 of an intermediate separation member 72, and the third and fourth ridges protrude downward from a reverse (lower) surface of the separation member 72.

The upper channel 46a is for receiving the upper row of teeth of a user. The lower channel 46b is for receiving the lower row of teeth of a user. The separation member 72 may form a biting surface. Optionally bristles or other cleaning elements may be provided extending into one or both of the tooth receiving channels 46a, 46b, for example extending from faces of the protruding ridges which face into the channel. These may brush or clean teeth when received in the channel.

By way of example, the separation member may form the support body 12 of the examples discussed above, and discussed further herein, having a support surface 13 for supporting particles of the agent to be delivered, and with the generated field for guiding particles along said surface 13. Thus, in the example of Fig. 3, the biting surface of the support member 72 may form the support surface 13, and particles may be guided along this support surface 13 to one or more desired locations around the upper and lower rows of teeth (during operation).

In other examples however, particles may be guided along a different surface of the device, e.g. a surface or face of one or more of the protruding ridges 14a, 14b, 14c, 14d. For example, the particles may be guided along an outer surface of one of the ridges, meaning a surface exposed in the mouth (in use) and facing away from the channel 46a, 46b.

The field generator 16 may be integrated in the separation member 72 in some examples.

There are different ways of inducing forces on the susceptible particles 22, depending upon the particular electric or magnetic properties of the particles the device 8 is to be used with.

According to one advantageous set of embodiments, the field generator 16 and/or control signal may be configured to generate a non-uniform field 20 (non-uniform field strength) having a field strength gradient in one or more directions, for guiding the particles 22 along a direction of the gradient.

A non-uniform field such as this can be used to move electrically polarizable or magnetisable particles, or electrically polarized particles for example. A polarized or polarizable particle will move along the direction of the field strength gradient due to the non-uniform force experienced at each pole. Thus the field can be configured so as to exhibit field strength gradients which define one or more gradient paths leading toward locations where the particles are desired to be delivered.

Alternatively to guiding particles using field strength gradients in a non-uniform field, electrically charged particles or magnetic particles can be guided along a direction of field lines of a field, where the field can be uniform or non-uniform. The particles will be guided along the vector direction of the field (either positively or negatively, depending upon the charge of the particle, in the case of electrically charged particles).

In addition to variations in the form of the generated field, as discussed above, embodiments can also be broadly grouped into electric field solutions and magnetic field solutions.

Examples of electric field solutions will first be described in more detail.

According to a one or more embodiments, the field generator 16 and/or control signal may be configured to generate a non-uniform electric field (or electromagnetic field), having a field strength gradient in one or more directions, for guiding polarizable particles 22 along a direction of the gradient. It also can be used to guide charged particles along a direction of the field lines.

A non-uniform AC (alternating) electric field can be used to move electrically polarizable or polarized particles by means of the dielectrophoresis (DEP) effect.

An example is schematically illustrated in Fig. 4. For illustration, this embodiment is again shown in the form of a mouthpiece device, but any of the other implementation options discussed in this disclosure may alternatively be used. Again, the controller 10 is not shown, for ease of illustration, but is included operatively coupled to the electrodes 18a, 18b of the field generator portions 16a, 16b.

The field generator 16 comprises a plurality of field generation arrangements or generator portions 16a, 16b, each comprising a respective pair of electrodes 18a, 18b separated by a space (extending from one electrode to the other), for generating an electric field 20 in the space. The field may be for imparting a force on the particles 22 to guide movement of the particles through the space, form one point to another. The particles thus may in general move in a direction toward one of the electrodes 18a, 18b and away from the other of the electrodes 18a, 18b.

To facilitate the non-uniform electric field 20, the electrodes 18 of each pair differ in respective size. For example, the first electrode 18a in Fig. 4 is longer than the second electrode. It may be longer in a dimension perpendicular the direction of the separation between the pair of electrodes for example.

Thus an asymmetric pair of electrodes is provided. By providing one electrode 18a which spans a larger distance than the other 18b along a direction perpendicular their separation, this results in a field which is more spatially spread on one side of the separation space than the other, with the result that there is a net force toward the smaller electrode.

This effect is illustrated in Fig. 5 which shows an example pair of electrodes 18a, 18b, separated by a space, D. One electrode 18a is negatively charged, the other 18b is positively charged, thereby inducing an electric field between them. The first electrode 18a is longer than the second 18b in a dimension perpendicular the direction of separation, D, between the electrodes. This has the effect that for a polarized or polarizable particle 22 located within the field, there is a net force on the particle toward the smaller electrode 18b, along the direction of separation between the electrodes. This is because in the non-uniform field, the particle is not uniformly polarized, since different parts of the particle are in field regions of differing field strength.

Where the particle is an electrically polarizable particle, and the pair of electrodes is driven with an alternating potential to create an alternating field, this effect is known as the dielectrophoresis (DEP) effect.

In this effect, the electric field 20 polarizes the particle 22, so that the poles experience a force along the field lines, which can be either attractive or repulsive according to the orientation on the dipole. Since the field 20 is non-uniform, the pole experiencing the greatest electric field will dominate over the other, and the particle will move in that direction.

It is noted that providing electrodes of different lengths is not the only way to create a spatially non-uniform field. An alternative means for instance is to provide a pair of electrodes of the same length, spaced from one another, but disposed at an oblique angle with respect to one another.

In some examples, each electrode 18a, 18b of the pair may have a respective electrically chargeable or active area for generating said electric field 20 in the space between the pair when electrically charged, and wherein the electrically chargeable or active areas of the two electrodes may differ in size. Thus the parts of the electrodes 18a, 18b which become charged and contribute to the field may differ in area between the electrodes. They may additionally or alternatively differ in dimensional extension along a direction perpendicular to a direction of the separation between the electrodes.

More generally, any electrode arrangement can be used in which the potential gradient between the electrodes varies spatially.

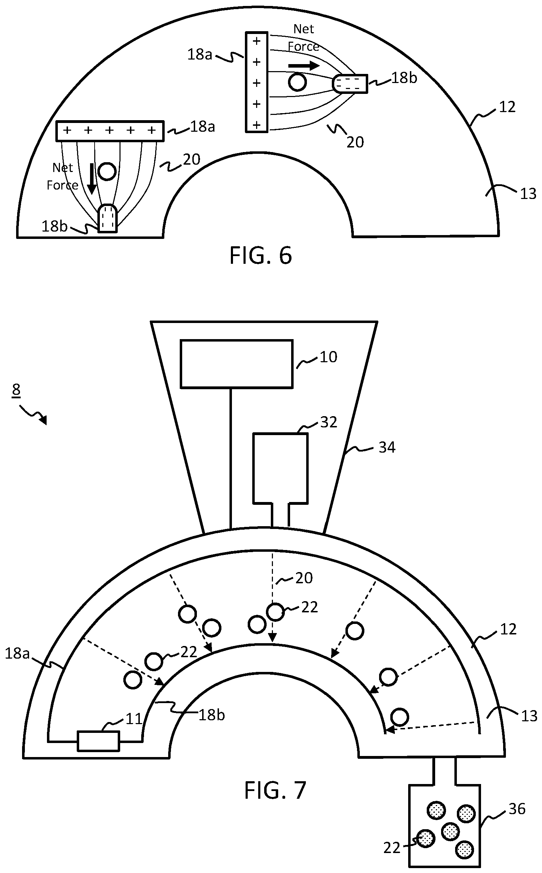

For example, Fig. 6 shows an example in which one 18a of the electrodes of each pair of electrodes is a flat plate electrode, and the other electrode 18b has a different, round-ended shape, so that its electrically active area extends around side surfaces of the electrode. It is shorter in a direction perpendicular the direction of separation of the electrodes. In addition, it has an electrically chargeable area contributing to the field which is smaller than that of the first electrode 18a. The second electrode 18b effectively forms a node electrode, while the first electrode 18b forms an elongate electrode.

A non-uniform electric field arrangement may be used with a range of different types of particles 22. The particles should be electrically polarized or polarizable, and preferably are polarizable. Suitable particles include for example living cells, but also cell-like particles with a membrane surrounding a liquid center which comprises the active agent (e.g. liposomes).

One advantageous set of embodiments proposes to use electrically polarizable capsules having oral treatment agents contained therein, the electrically polarizable capsules providing the particles susceptible to the field. These capsules can be moved by means of the non-uniform electrical field to the desired locations in the mouth. The active agent therein may then be deployed at those locations. This might happen without further interaction from the device, e.g. the capsules are water soluble and break down due to interaction with saliva, or it may be triggered by an activation stimulus generated by the device.

Furthermore, in embodiments utilizing a non-uniform field in combination with polarized or polarizable particles, the movement of the particles is dependent on a vector direction of a gradient in field strength, not a direction of the field lines. As a result, the movement in a given direction can be induced with an AC, alternating, field, despite the cyclically varying field direction.

As a result of this, according to one or more embodiments, the controller 10 (not shown in Figs. 4-6) may be adapted to provide an alternating drive signal between the electrodes 18a, 18b of each pair of electrodes, to thereby induce a (non-uniform) alternating field between each pair of electrodes. If the alternating field is driven at a radio frequency (RF), by providing an RF alternating voltage or current source across the electrodes, this has the secondary benefit that the RF field can perform an oral cleaning function at the same time as performing the function of guiding movement of the particles.

By way of non-limiting example, RF fields may be used with a frequency in the range of 3 kHz - 300 GHz, for example 10 kHz -100 MHz. A preferred frequency range may be 500 kHz - 30 MHz. RF fields with frequencies in the order of 1MHz, for example between 0.5 MHz and 1.5 MHz, have been found to be particularly effective in inducing movement of polarizable particles using the dielectrophoresis (DEP) effect.

By way of illustration, further example embodiments in which an electric or electromagnetic field is generated between two electrodes are illustrated in Figs. 7, 8 and 9, and these will be discussed below.

Fig. 7 shows an example device in the form of a cleaning or treatment mouthpiece unit. The device comprises a support body 12 having a support surface 13, the support body for being received in an oral cavity of a user. The support body is coupled to a handle portion 34 which houses a controller 10. The handle portion in this example is shown as comprising a local reservoir 32 for holding particles of an active agent or substance for delivery in the mouth. It is fluidly connected to a deployment surface 13 of the device, e.g. via one or more fluid passages, to enable particles to be received on the surface in use ready for delivery.

Additionally or alternatively, the device may be adapted to releasably couple or dock with an external reservoir 36, such as a bottle or other receptacle. When docked, particles can be loaded from the reservoir onto the support surface 13 ready to de delivered in the mouth.

In the example of Fig. 7, the device comprises a first 18a and second 18b arcuate electrode, the electrodes spaced from one another radially (along a radial dimension of the arcuate or semi-annular support body). The first electrode 18a extends proximal to, and follows the contour of, an outer circumferential edge of the body 12. The second electrode 18b extends proximal to and follows the contour of an inner circumferential edge of the body 12.

The two electrodes are connected to a RF driver or voltage source 11, and supplied with opposing polarity voltages. The drive signal may be static, so that a static field is generated, or an alternating signal may be applied across the electrodes, so that potential is oscillated back and forth between the electrodes.

It is noted that although the electrodes 18a, 18b extend parallel (concentrically) with one another in Fig. 7, this is not essential, and in other examples, non-parallel arcuate electrodes may be provided, which may provide a greater non-uniformity in the generated field.

A separate RF driver 11 and controller 10 are shown in Fig, 7. For example, the controller controls the drive scheme of the driver. However, in other examples, the controller itself may provide the drive signal source 11, so that a separate driver is not needed. In this case all drive signals may be generated by the controller in the handle for example. The pair of electrodes 18a, 18b, in combination with the drive signal source, provides a field generator 16.

When the electrodes 18a, 18b are driven with an alternating field, electrically polarizable particles 22 are driven to migrate along field gradient lines, toward the smaller, inner electrode 18b. The field gradient direction in this case is the radial direction, toward the second electrode, and the gradient is uniform along a circumferential direction. As a consequence, particles are induced to collect evenly along the length of the second electrode.

Alternatively, the electrodes may be driven to generate a static DC field, in which case charged particles may be induced to follow a direction of the field lines. The direction of the field lines, and thus the direction of particle migration in this case, will depend upon the polarities of the two electrodes 18a, 18b, and thus can be reversed by reversing the polarities. In this example, the direction of the field lines is also radial (either radially outward or radially inward).

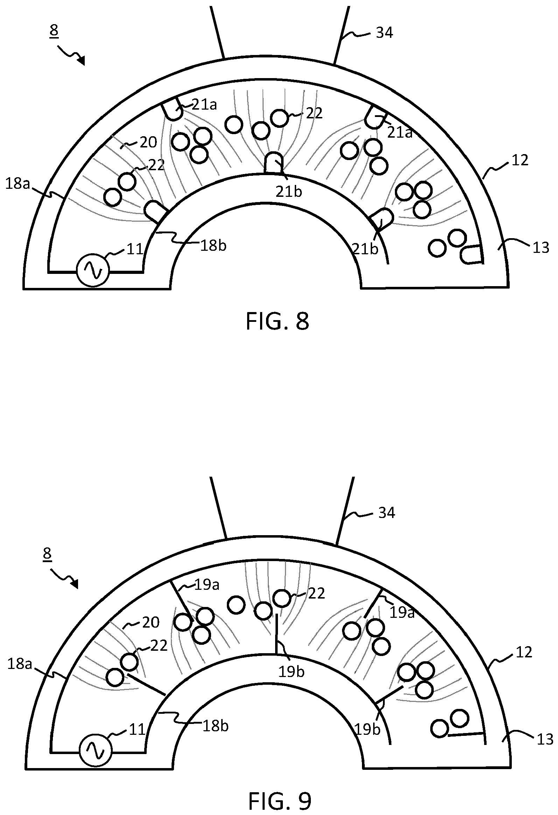

Fig. 8 shows a further example oral cleaning device 8 according to one or more embodiments. This embodiment may be the same in all respects as that of Fig. 7 apart from the additional provision to each of the first 18a and second 18b electrodes of radially protruding electrode nodes 21. The first electrode 18a comprises a first set of nodes 21a, spaced along the length of the electrode, facing toward the second electrode 18b, and the second electrode 18b comprises a second set of nodes 21b, spaced apart and facing the first electrode. The first and second sets of nodes are circumferentially offset from one another, so that each node radially faces a node-free space on the other opposing electrode.

This pattern results in a non-uniform field, but wherein the field strength gradients guide particles 22 toward the locations of the nodes 21a, 21b, Thus the nodes allow for defining of more localized particle collection points across the support surface 13 (when polarizable particles are used and an alternating field induced), permitting guidance of particles to more specific locations in the mouth when in use.

Fig. 9 shows a further example oral cleaning device 8 according to one or more embodiments. This again may be similar in all respects to the embodiment of Fig. 7 (and Fig. 8) apart from the configuration of the first and second electrodes. In this example, the first 18a and second 18b electrodes are provided in the form of arcuate interdigitated electrodes. The first 18a and second 18b electrodes each comprise a set of protruding electrode fingers 19a, 19b. These effectively perform the same function as the nodes 21 of Fig. 8, in providing more spatially defined particle collection points. When the electrodes 18a, 18b are driven with an alternating drive signal 11, to create an alternating field, the field strength gradients are directed toward the electrode fingers 19a, 19b thereby guiding polarizable particles 22 toward the electrode fingers.

As with the nodes of Fig. 8, the first electrode 18a comprises a first set of fingers 19a, spaced along the length of the electrode, facing toward the second electrode 18b, and the second electrode 18b comprises a second set of fingers 19b, spaced apart and facing the first electrode. The first and second sets of electrode fingers are circumferentially offset from one another, so that each finger radially faces a free space on the other opposing el ectrode.

The advantage of electrode fingers over nodes is that field strength gradients are directed toward their distal (free) ends, and the finger elements 19 can be made as long (in the radial direction) as desired. Thus it is possible to more flexibly define the locations for particle collection along the support surface 13, and thus in the mouth. For instance, in the example of Fig. 9, each of the electrode fingers 19 extends to a point approximately equidistant between the first 18a and second 18b electrodes, allowing for particle collection along a linear circumferential region near the middle of the support body 12.

In each of the examples of Figs. 7-9 above, the arcuate electrodes 18a, 18b are shows as extending parallel to one another (in a concentric arrangement). However this is not essential and in other examples, non-parallel arcuate electrodes may be provided, which may provide a greater non-uniformity in the generated field.

Furthermore, although in each of the examples of Figs. 7-9, the device is illustrated as including a separate field driver element 11 comprised by the support body, this is not essential. For example, a controller may be included in the handle in each embodiment, the controller arranged to generate the drive signal for the electrodes, so that all drive electronics are confined to the handle (which may preferably remain outside of the mouth during use).

Above have been discussed a number of examples in which electric or electromagnetic fields are generated.

However, in further embodiments, the field generator 16 and/or control signal may be configured to generate a magnetic field (uniform or non-uniform).

In this case, the field generator 16 may comprise one or more electrically conductive elements 18 which are arranged for carrying an electrical current. The controller 10 is adapted to supply a drive signal to the conductive elements to drive a current through the elements to generate a magnetic field in the space. The field may be for guiding magnetic particles along a direction of field lines of the field for example.

The conductive elements 18 may be electrodes, wires, or coils for example.

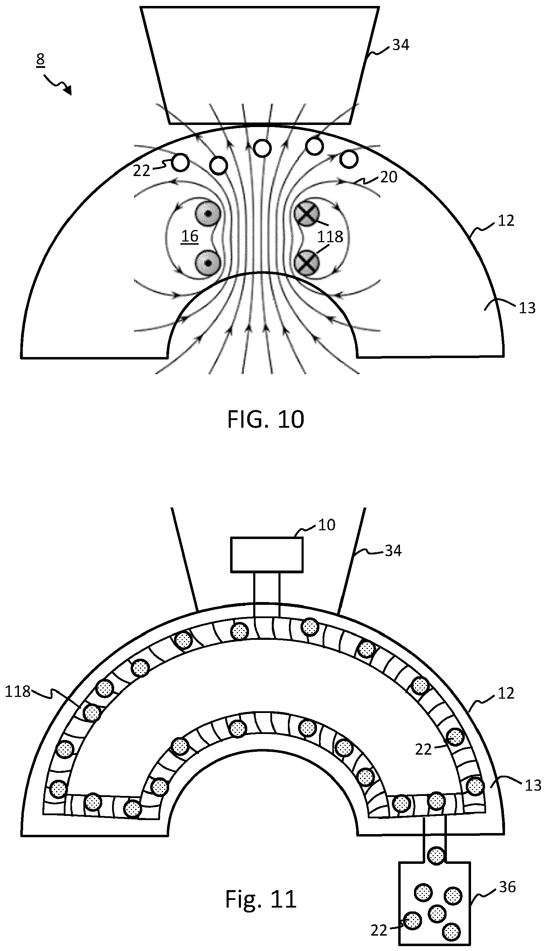

An example is schematically depicted in Fig. 10 for example. For illustration, this example is also shown in the form of a mouthpiece unit comprising a support body 12 for receipt in the oral cavity, the support body having a support surface 13. In this example, the field generator 16 comprises an conductive wire coil 118 which is arranged to receive an DC or AC current drive signal from the controller 10 (not shown), which current thereby induces a magnetic field 20 along a direction of an axial core of the coil, as illustrated in Fig. 10. The coil 118 is arranged so that the induced field 20 has field lines which extend from one circumferential side of the support body 12 support surface 13 to the other circumferential side. In this way the particles may be moved from one location in the oral cavity (mouth) to another.

Magnetic particles 22 are thereby guided along the vector direction of the field 20 which is generated.

The mouthpiece support body 12 may for example have a structure shaped to receive the upper and lower teeth in respective channels and shaped to conform to the shape of the interior of the mouth. Thus moving the particles over the surfaces of the mouthpiece, enables homogeneous distribution of magnetic particles around the mouth or around the teeth. For example, the mouthpiece may have a structure substantially as shown in Fig. 3 and described in detail above.

The device 8 further includes a handle 34 portion which may for example take the form as described above in relation to Fig. 2 or Fig. 7. A reservoir of particles may be included in the handle portion 34, and/or a separate reservoir may be provided in some examples for loading the device with particles of an oral treatment agent for deployment in the mouth, as discussed above. An external reservoir, in combination with the support body 12, controller 10 and field generator 16 may form an oral treatment system.

The controller 10 (not shown) may also be integrated in the handle 34 in some examples.

The structure of Fig. 10 represents just one example, and other arrangements of a device utilizing a magnetic field are also possible. For example, the device may take a different form from a mouthpiece. It may for example be in the form of a different type of oral care device such as, by way of non-limiting example, a toothbrush, an oral irrigation unit, or a flossing device. More than one coil 118 may be provided in further examples, such that a net magnetic field can be generated composed of a plurality of local magnetic field regions, each generated by one of the coils. This may enable more fine-grained shaping of the generated overall magnetic field. Additionally, the field generator may comprise one or more conductive elements in a different form to a coil, e.g. a loop or straight wire. A straight wire generates a circular magnetic field around it for example.

Fig. 11 schematically depicts a further example oral care device 8 according to one or more embodiments. This example is also shown in the form of a mouthpiece unit comprising a support body 12 for receipt in the oral cavity, the support body having a support surface 13. In this example, the device comprises a coil 118 whose axial extension is arranged to extend around an outer perimeter of the support body. In particular, the conductive coil 118 has one section which extends adjacent, and follows the outline of, an outer circumferential edge of the support body 12 and a further second section which extends along the inner circumferential edge of the support body. These two sections are joined by further coil sections at each end.

The coil 118 may be integrated, e.g. embedded, in the support body 12, beneath the support surface 13, shielded from the teeth.

A controller 10 is provided, which may for example be integrated in a handle 34 portion of the device 12. The controller 10 is arranged to drive the coil with a DC or AC current, preferably a DC current. This thereby induces a magnetic field having vector field lines which run along the direction of the axial core of the coil 118. There is thus induced a magnetic field which has field lines following a path extending around a periphery of the support surface 13. Magnetic particles located on the support surface will thus be guided along a path around the periphery of the support surface, and distributed to locations of the mouth or teeth adjacent these regions.

By way of one advantageous example, the support body 12 of Fig. 11, with the coil field generator arrangement shown, may be used as the separation element 72 or biting surface of the mouthpiece unit shown in Fig. 3 and described above. If implemented in this way, magnetic particles may be advantageously distributed in use around the outer edges of at least the upper tooth-receiving channel 46a. For example, particles may be guided around the joining edge between the first ridge 14a and the separation element 72 and the second ridge 14b and the separation element. This means that, in use, when the upper row of teeth is received in the channel 46a, the particles of the treatment agent are distributed around the buccal and lingual tooth sides and potentially also the interdental spaces. Thus, distribution of particles evenly around the teeth can be achieved in this example.

A similar effect may likewise be created in the lower channel 46b if the coil is embedded in the separation element 72 since the field penetrates to both upper and lower surfaces of the separation element and thus may also guide particles around the under-side surface of the separation element, and so around the teeth received in the lower channel 46b. In other words, particles can be moved along the upper and lower surfaces of the separation element 72 or support body 12.

In some examples, the coil 118 may be embedded beneath the support surface 13. In further examples, the coil may protrude partially above the surface (but shielded from contact with teeth).

As in previous examples, a local or external 36 particle reservoir may be provided for supplying particles 22 of a treatment agent to be deployed. Fig. 11 depicts by way of example an external reservoir 36 in the form of a bottle arranged to fluidly couple with the support body 12 for loading the body with particles for deployment. A flow path may be provided permitting transport of received particles to the support surface 13.

Any type of magnetic or magnetically polarizable particles may be used with this set of embodiments. There may be used magnetic or magnetisable capsules which contain a treatment agent therein.

An advantageous set of embodiments proposes to use catalytic antimicrobial robots (CAR) as the treatment agent to be deployed by the device. These exploit iron oxide nanoparticles with dual catalytic magnetic functionality to (i) generate bactericidal free radicals, (ii) break down the biofilm exopolysaccharide (EPS) matrix, and (iii) remove the fragmented biofilm debris via magnetic field gradient-driven robotic assemblies in a controlled manner, preventing biofilm regrowth.

CARs are magnetic particles and thus can be induced to move using a magnetic field. They may move along the directions of magnetic field lines for example. In particular, CARs contain iron oxide nanoparticles (NP) which can be moved using the induced magnetic field. By creating a field which is distributed across the support surface 13, a homogeneous distribution of the CARs can be created in e.g. a mouthpiece. This will improve the removal of biofilm on dental surfaces for example.

Structure, formation, use and deployment of CARs is described in detail in the paper: Catalytic antimicrobial robots for biofilm eradication, Hwang, G et al, Science Robotics, 24 Apr 2019, Vol. 4, Issue 29, eaaw2388, DOI: 10.1126/scirobotics.aaw2388.

By way of one example, CARs may be used suspended in a solution containing hydrogen peroxide and enzymes. This may be guided using the induced magnetic field to locations in the mouth at which a biofilm is present. The CARs have the effect of generating bactericidal free radicals on-site which will firstly kill the bacteria in a biofilm. This will consequently degrade the matrix (EPS) of the oral biofilm. Using an applied magnetic field, the CARs then work to remove the degraded biofilm and biofilm regrowth is prevented.

In accordance with one or more embodiments, the coil may be driven with an RF frequency AC current such that an RF alternating magnetic field is generated. This advantageously permits the magnetic field to perform an RF cleaning function at the same time as guiding movement of the particles.

In some examples, the oral cleaning device may comprise a plurality of bristles for cleaning teeth. For example the device may be a toothbrush device, or may be a mouthpiece device incorporating bristles in one or both tooth-receiving channels. The magnetic particles may be guided across a surface carrying the bristles, and distributed across the bristle field, to thereby permit application of the particles on the teeth when the bristles rub over the teeth.

In some examples, the iron oxide particles may be provided mixed with an agar gel to form certain 3D shapes and 3D formations. These formations may then be guided around the mouth based on application of magnetic or electromagnetic fields.

As discussed above, in accordance with one or more embodiments, an alternating RF field can be generated and used to provide an oral cleaning function.

In accordance with any of the embodiments discussed above for example, the controller 10 may be adapted, in at least one mode, to generate a control signal configured to cause the field generator 16 to generate an RF alternating field (which may be electric, magnetic or electromagnetic), for performing an oral cleaning function when the field generator is received in an oral cavity of a user.

For example, the field generator 16 may comprise one or more conductive elements 18 as discussed above, and the controller 10 may supply a control signal in the form of an RF alternating drive signal to or between the one or more conductive elements.

In this way, the same components of the field generator 16 which are used to manipulate the positioning of the particles 22 are also used to generate an RF field for cleaning.

In some examples, the first field 20 (for guiding movement of the particles) may itself be an RF alternating field. Thus, the field which induces movement of the particles 22 to the desired location may advantageously also performs an RF oral cleaning function due to its RF frequency alternation. The RF cleaning function may occur simultaneously to the particle movement function.

Where the field 20 has a non-uniform field strength for moving polarizable particles, this is straightforward. The alternation of the field direction during each cycle does not change a direction of the field strength gradient along which the particles are induced to move.

Where the particles to be moved are charged particles, where the field may be uniform or non-uniform, it may be necessary to alternate a field strength or amplitude of the field between a forward and backward phase of the cycle, so that there is a net force in one direction.

In further examples, the first field (for guiding movement of the particles) may not itself be an RF field, or may be an RF field, but may not perform an RF cleaning function.

In accordance with one or more embodiments, the controller 10 may be selectively adjustable between at least two different modes:
a particle delivery mode in which the controller 10 supplies a control signal to the field generator 16 for generating a field for guiding particles 22 susceptible to the field to a desired one or more locations in a space; and
a cleaning mode in which the controller 10 supplies a control signal to the field generator 16 for generating an RF field in a space for performing an oral cleaning function.

The field 20 generated in the particle delivery mode may be an RF field, or may not be an RF field (e.g. it may be a DC field). In some cases, the field generated in the particle delivery mode may be an RF field, having a different RF frequency to the RF field of the cleaning mode.

RF fields may be used with frequencies for example anywhere in the range of 3 kHz - 300 GHz, for example 10 kHz-100 MHz. A preferred frequency range may be 500 kHz - 30 MHz. The RF field may, by way of non-limiting example, have a total output power in a range from 0.1 W to 15W, for example 0.1 W to 10W.

In accordance with one or more embodiments, the device may be configured with functionality for triggering activation of delivered particles 22. This activation may be a chemical or physical reaction process, or a process of breaking down particles to trigger release of their contents. This step may be performed subsequent to moving the particles to the desired locations in the mouth.

Thus in accordance with one or more embodiments, the device 8 may further comprise a particle activator adapted to generate a physical stimulus for stimulating an activation event of the susceptible particles. The particle activator may be composed of the same or different components to the field generator. Thus, the particle activator may be provided by the field generator 16 (e.g. one or more pairs or electrodes, or one or more coils) in some examples, or may be a formed by a separate set of one or more components.

The stimulus may, by way of non-limiting example, include at least one of: a generated field (electrical and/or magnetic stimulus), a heat stimulus, and an acoustic stimulus.

In accordance with one or more embodiments, the device 8 may further comprise a cleaning stimulus generator, for generating a physical stimulus for stimulating breakdown or removal of a biofilm from a surface within an oral cavity of a user. Again, the cleaning stimulus generator may be composed of the same or different components to the field generator 16.

In accordance with one or more embodiments, the controller 10 may be adapted, in at least one control mode, to execute a particle delivery control scheme.

The control scheme comprises at least a first step of supplying the control signal to the field generator 16 (e.g. one or more pairs or electrodes, or one or more coils) to guide the particles 22 to the desired location. This is a particle deployment step.

The control scheme further comprises at least a second (subsequent) step of supplying a second control signal to the particle activator to create a stimulus for activating the particles 22. This is a particle activation step.

Optionally, the control scheme may further comprise at least a third step of supplying a third control signal to the cleaning stimulus generator to generate a cleaning stimulus for stimulating breakdown or removal a biofilm residue on an oral surface (e.g. at the location of the activated particle(s)).

Where the particle activator is provided by the field generator 16, the first and second control signals may differ in field characteristics such as frequency and/or amplitude.

Where the cleaning stimulus generator is provided by the field generator 16, the first and third (and preferably second) control signals may differ in field characteristics such as frequency and/or amplitude.

One particular example of this particle deployment control scheme, as implemented for an embodiment in which the field is an electric field (or electromagnetic field), may be as follows.

In a first (particle deployment) step, treatment agents are delivered to desired locations in the mouth. The treatment agents may be topical agents such as fluoride, whitening solutions, mouthwash, specialized toothpaste, gel for mouthpieces or any other type of topical agent. The topical agent is incorporated in advance into electrically polarizable particles or capsules which contain the topical agent.

The field generator 16 may be controlled to generate an RF alternating electric (electromagnetic) field in a space, the field having a non-uniform field strength. This induces a force on the polarizable capsules which guides them to a particular location of treatment. For the particle deployment step, in which the particles are moved, the RF field may be driven to alternate at a first RF frequency.

Once the capsule has been moved by the RF field to the treatment area, the capsule is activated in an activation step to thereby release the topical agent inside. This can be done through a variety of different mechanisms, such as heat stimulus, acoustic stimulus or electromagnetic stimulus. By way of one example, the field generator 16 may be controlled to generate a further RF field at a second frequency to induce RF heating.

The alternating frequency used for heating is typically higher than the frequency for cleaning or for movement. For example, suitable frequencies for heating may be in the order of 1 MHz-1 GHz.

Heat sensitive liposomes are an example of such stimulus-activated particles containing the topical agent. After delivery using a first intensity RF field, the liposomes can be opened to release the topical agent using a higher intensity RF field, for heating the particles, thereby releasing the contents locally, e.g. on a tooth surface or between teeth.

An optional third step comprises controlled removal of degraded biofilm. This can be achieved by driving the field generator to generate an RF field at a third RF frequency. This may be a frequency from the same range of frequencies as discussed above for the RF field used for the cleaning function.

Above have been described a number of embodiments comprising actively driven field generators. However, as mentioned above, passive field generators are also possible in accordance with further embodiments.

For example, according to one set of embodiments, the field generator may comprise a permanent magnet arrangement. The field generator generates a static magnetic field based on use of the permanent magnetic arrangement, the static magnetic field for guiding magnetic particles along a direction of field lines of the field, or along a direction of field strength gradients. The magnetic particles may include any kind of magnetic particle, including any of those discussed above, such as CARs.

The generated magnetic field has vector field lines which may be shaped for guiding magnetic particles along one or more paths or in one or more directions.

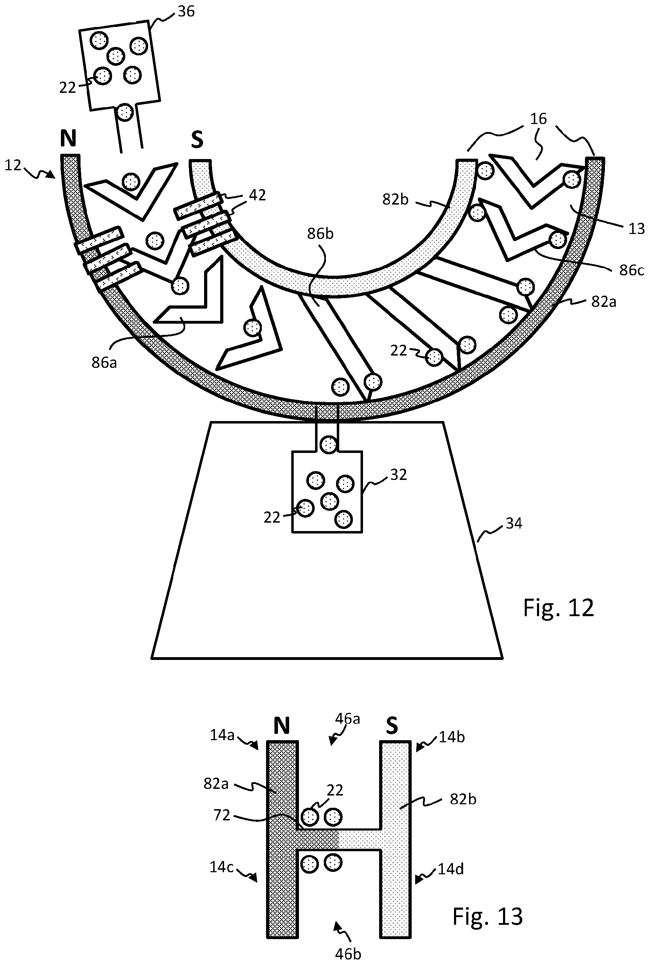

An example oral care device according to this set of embodiments is shown in Fig. 12.

This example is in the form of a mouthpiece unit. The device comprises an arcuate support body 12 having a support surface 13. The device comprises a passive field generator 16 which comprises a permanent magnet arrangement 82a, 82b formed of a first permanent magnet portion 82a, and a second permanent magnet portion 82b. In this example, each of the magnet portions is a separate magnetic element, however in other examples, the portions may be sections of a single unitary permanent magnet.

The first 82a and second 82b magnet portions form opposing magnetic poles (North and South respectively). In the illustrated example, each magnet portion takes the form of a magnetic strip or element having a curved or arcuate shape, and with the first magnet portion 82a arranged extending along and around an outer circumferential edge of the support surface, and the second magnet portion 82b extending along and around an inner circumferential edge of the support surface. The two magnetic elements 82a, 82b are radially spaced from one another, with therefore a static magnetic field extending radially over the support surface 13 between the first 82a and second 82 magnetic portions.

The field generator 16 further includes a set of (passive) field shaping elements 86a, 86b located in the region between the first 82a and second 82b magnetic portions. Each field shaping element comprises a body formed of or comprising a magnetic material which is arranged to become magnetized in the permanent magnetic field between the first 82a and second 82b magnetic portions. Each field shaping element may be formed of or comprise a ferromagnetic material for example. For example, each may be formed of a magnetic material wire or plate. The geometry and shape of the field shaping element partially determines a direction in which magnetizable or magnetic particles are guided to move, e.g. a flow direction of magnetizable or magnetic particles. Each field shaping element may be located (e.g. embedded or recessed) beneath the support surface 13 in some examples, or may be disposed on the support surface, e.g. adhered or printed or engraved onto the support surface.

The shape of each field shaping element is configured to shape the magnetic field lines in its vicinity so as to define a magnetic field guidance path which guides particles along a particular trajectory or path when brought into the field. For example, when magnetized, each field shaping element generates a secondary magnetic field which shapes the resultant field in the region between the first 82a and second 82b magnetic elements.

Fig. 12 shows three examples of field shaping elements. For instance, in the example of Fig. 12, the device includes a first type 86a of field shaping element which has a pointed V-shape form, comprising two linear sections, e.g. lines or bars, which are angled obliquely toward one another. In this example, the two linear sections meet at an apex which points in a direction transverse the first 82a and second 82b magnet portions (i.e. a circumferential direction in Fig. 12). This shapes a magnetic field in its vicinity to have field lines which guide a magnetic particle (received at the open side of the V) along a path transverse the first and second magnetic portions, i.e. along a circumferential direction). For example, the first field shaping elements may guide particles received at the open side of the V along a line which bisects the V-shape, passing through the apex of the V. For example, the first field shaping elements may be positioned to guide particles along a central arcuate path between the first and second magnetic elements.

The device further includes a second type 86b of field shaping element, having an elongate shape, e.g. comprising an angled line or bar, which has a length dimension pointing in the direction of one or both of the magnetic portions 82a, 82b. This form of field shaping element shapes the magnetic field so as to guide particles 22 toward one of the magnetic portions 82a, 82b, i.e. along the direction of the permanent magnetic field lines between the magnetic portions, i.e. a radial direction in Fig. 12. This guides particles toward one of the edges of the support surface, e.g. for deployment on teeth of gums of a user located at said edge in use.

The device further includes a third type of field shaping element 86c, which has the same shape and geometry as the first type 86a (i.e. V-shaped), but is orientated with the apex of the V facing in the opposite direction, so that particles are received at the apex side of the V. In this orientation, the particles are guided away from the apex of the V, along the length directions of the two arms of the V. The element may be positioned such that particles are guided toward one or other of the magnet portions 82a, 82b for example.

The field shaping elements 86 are not essential and may be omitted in further examples, in which case magnetic particles introduced to the support surface will be guided to one of the magnetic portions 82a, 82b, along the radial field lines between these portions.

The device optionally further includes a plurality of bristles 42 formed in bundles which extend inward to the region between the first 82a and second 82b magnetic portions, e.g. into a tooth-receiving channel formed between them.

As in previous examples, a local 32 and/or external 36 particle reservoir may be provided for supplying particles 22 of a treatment agent to be deployed.

Fig. 12 depicts by way of example an external reservoir 36 in the form of a bottle arranged to fluidly couple with the support body 12 for loading the body with particles for deployment. A flow path may be provided permitting transport of received particles to the support surface 13. Fig. 12 also depicts an internal particle reservoir 32 integrated in a handle 34 of the device. This may take the form of the internal reservoir discussed above in relation to Fig. 12 for example. One, both, or neither of the reservoirs may be provided in different example embodiments.

In the passive magnetic embodiments shown in Figs. 12-13, an additional fluid flow force may be provided which is advantageous for guiding particles (more efficiently). Therefore, for example, the local reservoir 32 may be part of an active fluid actuator or pump (not shown in Figs. 12-15). Likewise, the external reservoir 36 may include means for providing actuation to induce fluid flow, e.g. by squeezing a tube by hand which will generate a shear force and a residual flow or using an active fluid actuator (again not shown in Figs 12-15).

By way of one advantageous example, the mouthpiece device of Fig. 12 may take the form of the mouthpiece device of Fig. 3, having an upper tooth-receiving channel 46a and lower tooth-receiving channel 46b. With reference to Fig. 3 and Fig. 12, the support body 12 of Fig. 12, may be used as the separation element 72 or biting surface of the mouthpiece unit shown in Fig. 3 and described above. If implemented in this way, magnetic particles 22 may be advantageously distributed in use along the upper and/or lower tooth receiving channel. For example, particles may be guided around the joining edge between the first ridge 14a and the separation element 72 and the second ridge 14b and the separation element. This means that, in use, when the upper row of teeth is received in the channel 46a, the particles of the treatment agent are distributed around the buccal and lingual tooth sides and potentially also the interdental spaces. Thus, distribution of particles evenly around the teeth can be achieved in this example.

In this manner, it is possible for example to apply a static magnetic field across the width of a tooth-receiving channel (e.g. from between the buccal to lingual tooth sides or vice versa). The field shaping elements may be provided in the biting surface 72 which effectively apply a superimposed magnetic gradient field to the static field (to tune the flow direction of the particles).

According to one set of examples, the first magnetic portion 82a may be integrated in, or carried by, the first protruding ridge 14a, and the second magnetic portion 82b may be integrated in or carried by the second protruding ridge 14b. This is illustrated in Fig. 13, which shows a cross-sectional view of an example device, taking the form and structure of Fig. 3 and incorporating permanent magnet portions 82a, 82b in the two protruding ridges 14a, 14b. Thus a static magnetic field is induced between the two ridges across the upper tooth-receiving channel 46a.

In the illustrated example, the first 82a and second 82b magnetic portions also extend so as to be integrated in the third 14c and fourth 14c protruding ridges, such that a static magnetic field is also created between the third and fourth ridges, across the lower tooth-receiving channel 46b. This has the effect of drawing magnetic particles 22 brought into the upper or lower channels across to one side of the tooth-receiving channel. When the upper and/or lower rows of teeth are received in the upper and/or lower channels, this means that particles are drawn to one side of the teeth. The magnetic field extends from one side of each row of teeth to the other, e.g. from the lingual to the buccal side or vice versa, thus guiding particles from one side of the teeth to the other.

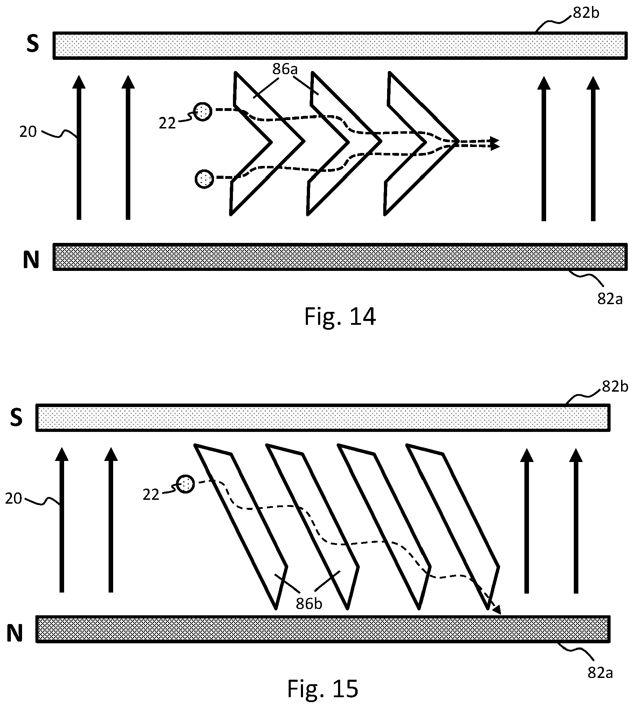

By way of further explanation, Figs. 14 and 15 further illustrate the shaping of the magnetic field in the region between the first 82a and second 82b magnetic field portions by means of field shaping elements.

Fig. 14 illustrates first 82a and second 82b magnetic portions with the first type 86a of field shaping element discussed above and shown in Fig. 12, having a pointed, apex (V) shape. A permanent magnetic field 20 exists extending between the two magnetic portions. Two example magnetic particles 22 are shown, and the resultant travel paths of the particles are indicated by dashed lines. The two particles begin at an open end of the V-shaped field shaping elements. As shown, the field shaping elements 86a shape the magnetic field so as to guide magnetic particles along a path transverse the lengths of the magnetic portions 82a, 82b and the path tapering inward toward an apex of the field shaping element, e.g. toward a central line or path between the first 82a and second 82b magnetic portions in this example. The particles are thus guided to a narrower central flow path.

Fig. 15 illustrates first 82a and second 82b magnetic portions with the second type 86b of field shaping element discussed above and shown in Fig. 12, having an elongate, bar or plate shape which has a length which points in a direction toward one or both of the magnetic portions at an oblique angle. In this case, a magnetic particle 22 is guided along a path transverse the lengths of the magnetic portions 82a, 82b and toward one of the two magnetic portions. Thus the particles are guided to one edge of the region between the magnetic portions.

As discussed above, in accordance with various embodiments, the field generator 16 may be carried by or integrated in a support body 12, the support body for receipt in an oral cavity of a user, for generating a field in the oral cavity for guiding the particles 22 to a desired one or more locations in the oral cavity. In the case of an active field generator, in some examples the field generator may comprise only passive parts (e.g. electrodes or coils), while the drive electronics are all located in a further portion of the device which is not designed for receipt in the oral cavity, e.g. a handle portion.

As discussed above, in accordance with at least one set of embodiments, the support body 12 may be in the form of a mouthpiece unit, and the mouthpiece arranged to fit into the mouth of a user with surfaces of the mouthpiece overlying or facing surfaces of the mouth.

The body of the mouthpiece may describe a U-shaped contour, for instance a portion of an annulus.

Hence here, the device comprises at least a portion which can be fitted in the mouth and has a shape and structure designed to follow natural contours and morphology of the mouth. In this case, a relative position of different parts of this device body to parts of the mouth can be reliably assumed, allowing the device to efficiently deploy particles to predetermined locations in the mouth.

However alternative options are also possible, for example devices for aiding deployment at local areas, such as a particular one or more teeth, where the user positions the device at the location they wish to apply the deployment effect. Such devices may make use of a support body in the form of a head of a cleaning device such as a toothbrush or powered flossing device for instance.

For example, the device 8 may comprise a toothbrush with a head portion comprising the field generator 16, and further comprising bristles. The device may further comprise a body or handle portion which may incorporate a controller 10. The handle may further comprise a particle reservoir as discussed above.

The toothbrush device may further comprise a mechanical oscillation generator for oscillating the head portion to induce oscillation of the bristles. This may also be incorporated in the body or handle portion.

The device according to any implementation may include a handle or body portion which electrically and mechanically couples to the mouth-receivable support body 12 portion, and which integrates the controller 10.

By way of one non-limiting set of examples, the oral care device according to embodiments of the present invention may take any of the following forms:
- a toothbrush device, e.g. a powered toothbrush;
- an oral irrigator device;
- a flossing device;
- a combined brushing and flossing device; or
- a mouthpiece device, e.g. brushing mouthpiece device.

A further aspect of the invention also provides an oral cleaning system, comprising:
a device 8 in accordance with any example or embodiment outlined above or described below, or in accordance with any claim of this application, and
a substance for delivery in the mouth, the substance comprising particles 22 susceptible to the field.

According to one set of advantageous embodiments, the substance may comprise (electrically) polarizable capsules containing a treatment agent for delivery.

According to a further set of advantageous embodiments, the substance may comprise (ferro-, dia-, para-) magnetic particles such as for example catalytic antimicrobial robots (CAR).

A further aspect of the invention also provides a method for delivering particles 22 for a treatment function within an oral cavity of a user, the method comprising:
delivering a control signal to a field generator 16, to control generation by the field generator of an electric and/or magnetic field 20, the field for imparting a force upon particles 22 susceptible to the field, for guiding movement of the particles to a desired location in an oral cavity of a user when the field generator is received in said oral cavity.

The method may be performed with the field generator 16 received in the oral cavity (mouth) of a user.

Examples in accordance with a further aspect of the invention provide a computer program product comprising computer program code, the computer program code being executable on a processor or computer
wherein, when the processor or computer is operatively coupled with a field generator, the code is configured to cause the processor to perform a method in accordance with any example or embodiment outlined above or described below, or in accordance with any claim of this application.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. An oral care device (8) comprising:
a field generator (16) adapted to generate a first electric, electromagnetic and/or magnetic field (20) in a space;
the field for imparting a force upon particles (22) susceptible to the field, for guiding movement of the particles to a desired one or more locations in the space,
wherein the field generator is for operation in a mouth of a user, for guiding movement of the particles to a desired one or more locations in the mouth.

2. A device as claimed in claim 1, wherein the field generator (16) is carried by or integrated in a support body (12), the support body for receipt in an oral cavity of a user, for generating a field (20) for guiding particles (22) across or over one or more surfaces of the support body.

3. A device (8) as claimed in claim 1 or 2, wherein the field generator is arranged to generate a spatially non-uniform field, having a field strength gradient in one or more directions.

4. A device as claimed in any of claims 1-3, wherein
the field generator (16) is adapted to generate said first field responsive to an input control signal; and
the device further comprises a controller (10) adapted to generate the control signal, to control generation of the electric, electromagnetic and/or magnetic field in the space, the field for guiding movement of the particles to a desired one or more locations in the mouth.

5. A device (8) as claimed in claim 4, wherein the field generator (16) comprises one or more conductive elements (18), electrically coupled to the controller (10), and wherein the control signal is an electrical drive signal for electrifying the one or more conductive elements.

6. A device (8) as claimed in claim 5,
wherein the field generator (16) and/or control signal is configured to generate a spatially non-uniform electric or electromagnetic field, having a field strength gradient in one or more directions, for guiding polarizable particles along a direction of the gradient and/or charged particles along a direction of field lines,
wherein the one or more conductive elements include at least one pair of electrodes (18a, 18b) separated by a space, and optionally
wherein the electrodes of the pair are different sizes.

7. A device (8) as claimed in claim 5,
wherein the one or more electrically conductive elements (18) are for carrying an electrical current,
wherein the controller (10) is adapted to supply a drive signal to the conductive elements to drive a current through the elements to generate a magnetic field (20) in the space,
wherein the magnetic field (20) is for guiding magnetic particles along a direction of field lines of the field.

8. A device (8) as claimed in any of claims 4-7, wherein the controller (10) is adapted, in at least one mode, to generate a control signal configured to cause the field generator (16) to generate an RF alternating field, for performing an oral cleaning function when the field generator is received in an oral cavity of a user.

9. A device (8) as claimed in claim 8, wherein said first field (20) is an RF alternating field.

10. A device (8) as claimed in any of claims 4-9, wherein the controller (10) is selectively operable in at least two different modes:
a particle delivery mode in which the controller supplies a control signal to the field generator (16) for generating a field (20) for guiding particles (22) susceptible to the field to a desired one or more locations in the space; and
a cleaning mode in which the controller supplies a control signal to the field generator for generating an RF alternating field in the space for performing an oral cleaning function.

11. A device (8) as claimed in any of claims 4-10, wherein the device further comprises a particle activator adapted to generate a physical stimulus for stimulating an activation event of the susceptible particles, wherein the particle activator is composed of the same or different components to the field generator, and
optionally wherein the device further comprises a cleaning stimulus generator, for generating a physical stimulus for stimulating breakdown or removal of a biofilm from a surface within an oral cavity of a user, wherein the cleaning stimulus generator is composed of the same or different components to the field generator.

12. A device (8) as claimed in claim 11, wherein the controller (10) is adapted to execute a particle delivery control scheme, the control scheme comprising:
at least a first step of supplying the control signal to the field generator (16) to guide the particles to the desired location, and
at least a second subsequent step of supplying a second control signal to the particle activator to create a stimulus for activating the particles;
and optionally further comprising at least a third step of supplying a third control signal to a cleaning stimulus generator to generate a cleaning stimulus for stimulating breakdown or removal a biofilm residue on an oral surface.

13. A device as claimed in any of claims 1-3, wherein
the field generator comprises a permanent magnet arrangement for use in generating a static magnetic field in said space, the static magnetic field for guiding magnetic particles along a direction of field lines of the field.

14. A device (8) as claimed in any of claims 1-13 wherein the support body (12) is in the form of a mouthpiece unit, and the mouthpiece unit arranged to fit into the mouth of a user with surfaces of the mouthpiece overlying or facing surfaces of the mouth and/or teeth.

15. An oral cleaning system, comprising:
a device (8) as claimed in any of claims 1-14, and
a substance or agent for delivery in the mouth, the substance comprising particles (22) susceptible to the field.

16. A method for delivering particles (22) for a treatment function within an oral cavity of a user, the method comprising:
delivering a control signal to a field generator (16), to control generation by the field generator of an electric, electromagnetic, and/or magnetic field (20), the field for imparting a force upon particles susceptible to the field, for guiding movement of the particles to a desired one or more locations in an oral cavity of the user when the field generator is received in said oral cavity.

17. A computer program product comprising computer program code, the computer program code being executable on a processor or computer,
wherein, when the processor or computer is operatively coupled with a field generator, the code is configured to cause the processor to perform a method in accordance with claim 16.
